# EUROPEAN PATENT SPECIFICATION

(11) **EP 0 461 319 B1**
(45) Date of publication and mention of the grant of the patent: **24.05.1995**
(21) Application number: 90312607.6
(22) Date of filing: 20.11.1990
(51) Int. Cl.: A61F 5/03

(54) **Back support vest**
Weste zur Rückenhaltung
Veste de soutien du dos

(30) Priority: 11.06.1990 US 535686
(43) Date of publication of application: 18.12.1991
(73) Proprietor: DeWall, Terry L., Omaha Nebraska 68137 (US)
(72) Inventor: DeWall, Terry L., Omaha Nebraska 68137 (US)
(74) Representative: Coxon, Philip

(56) References cited:
- DE-A- 2 709 369
- US-A- 4 022 197

## Description

This invention relates to back support vests and more particularly to vests that provide support of the entire back area from the thoracic to the lumbosacral region.

Eight out of ten Americans experience a painful back episode at some time during their lives. One hundred million Americans have serious back problems, and over one-quarter million have back surgery each year.

Many devices are known that provide support to specific regions of the back. However, no previously known device provides effective support to the entire back area to maintain the normal curvature of the spine while engaging in various physical activities.

Those concerned with these and other problems recognise the need for an improved back support vest.

In DE-A-2,709,369 there is disclosed a back support vest as recited in the preamble to claim 1. US-A-4,022,197 discloses an appliance for supporting and/or protecting body portions comprising a pair of rigid sheet-like members adapted to engage predetermined body portions and coupled by elastic material.

It is an object of this invention to provide an improved back support vest.

According to this invention there is provided a back support vest, comprising a back-supporting section disposed to encircle the human body; means for releasably securing the back-supporting section in body-encircling form; and a pair of shoulder straps disposed to join front and rear lateral portions of the vest; characterised in that: the back-supporting section comprises: a lower back-supporting section of an elastic disposed to encircle the lumbar and lumbosacral region; and an upper back-supporting section of a non-elastic material attached to, and extending above, said lower section and disposed to encircle the thoracolumbar and thoracic region; and in that the vest further comprises: a reinforced section including multiple layers of non-elastic material attached to said vest and disposed to extend from the lower section into the upper section, said reinforced section being centered on and extending across the dorsolumbar region and extending upwardly therefrom; and a pocket at the rear of said vest and disposed to extend across the lower section, said pocket being adapted to selectively receive a support device.

Preferably, said means for releasably securing the ends of the lower section includes overlapping ends attached with a presence sensitive contact fastener.

Preferably, said means for releasably securing ends of the upper section includes overlapping ends attached with a pressure sensitive contact fastener.

The pressure sensitive contact fastener is preferably in the form of a narrow fabric woven in pile form, with uncut and cut loops, and a ribbon woven in velvet form, whereby the narrow fabric can engage the ribbon to be fastened thereto. A suitable such fastener is a velcro-type fastener.

The present invention may provide a back support vest including a lower back supporting section formed of elastic material that encircles the body in the lumbar and lumbosacral region, an upper back supporting section formed of non-elastic material that encircles the body in the thoracolumbar and thoracic region, and shoulder straps that join the front and rear lateral portions of the upper section. The vest also includes a reinforced section that spans the lower and upper section centering on the dorsolumbar region. The elastic material of the lower section may keep the reinforced section in tension to provide effective support. Additional support may be provided by pockets that releasably attach to the rear of the vest and are adapted to hold additional back support devices such as pillows, inflatables and moulded spinal orthoses. the vest and are adapted to hold additional back support devices such as pillows, inflatables and moulded spinal orthoses.

It is an advantage of the present invention that it provides a back support vest that simultaneously supports the entire back, lifts the chest to move the shoulders back to a released position, and maintains the natural curvature of the spine.

A further advantage of the invention is that it provides a back support vest that is washable and easy to maintain.

Still another advantage is that it provides a back support vest that is durable.

A still further advantage of the present invention is that it provides a back support vest that moulds to the wearer's body, is cool and comfortable, and can be worn under street clothes without being noticeable.

Reference is now made to the accompanying drawings in which:-
Figure 1 is a perspective view of the back support vest of the present invention showing the front side having overlapping ends with the Velcro-type fastener to secure the vest in body encircling position, and the adjustable shoulder straps;
Figure 2 is a perspective view showing the rear of the vest having the selectively detachable pockets secured by Velcro strips;
Figure 3 is a rear elevational view showing the detachable pockets in place;
Figure 4 is a front elevational view;
Figure 5 is a side elevational view;
Figure 6 is a top plan view;
Figure 7 is a bottom plan view;
Figure 8 is an exploded perspective view illustrating the positioning of supporting devices in the detachable pockets; and
Figure 9 is an exploded perspective view illustrating the rear of the vest in an open layed-out configuration, and showing the orientation of the supporting devices in the detachable pockets.

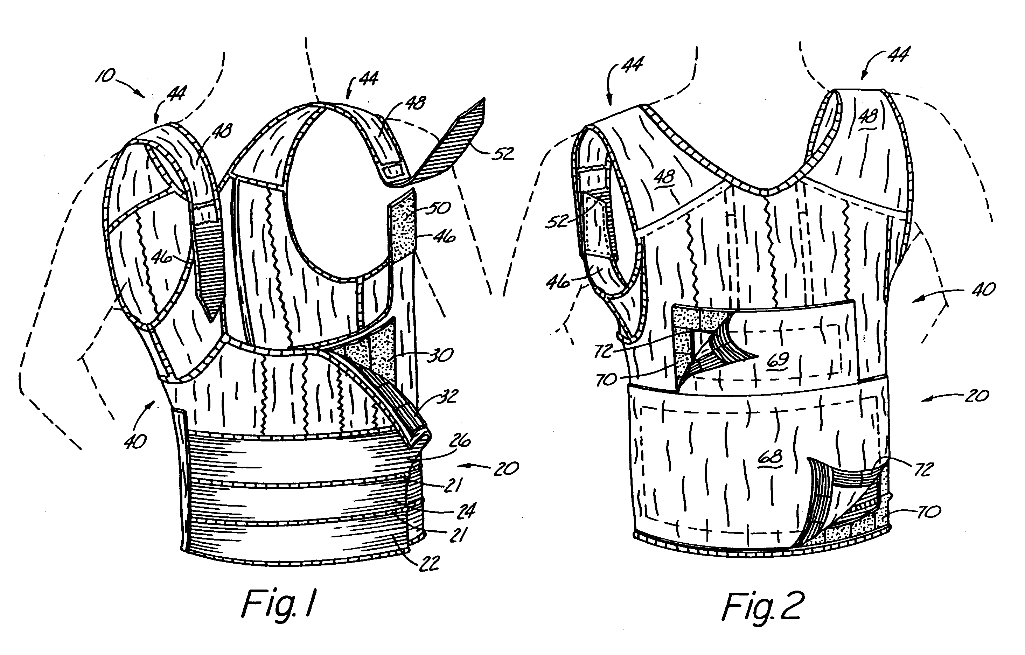

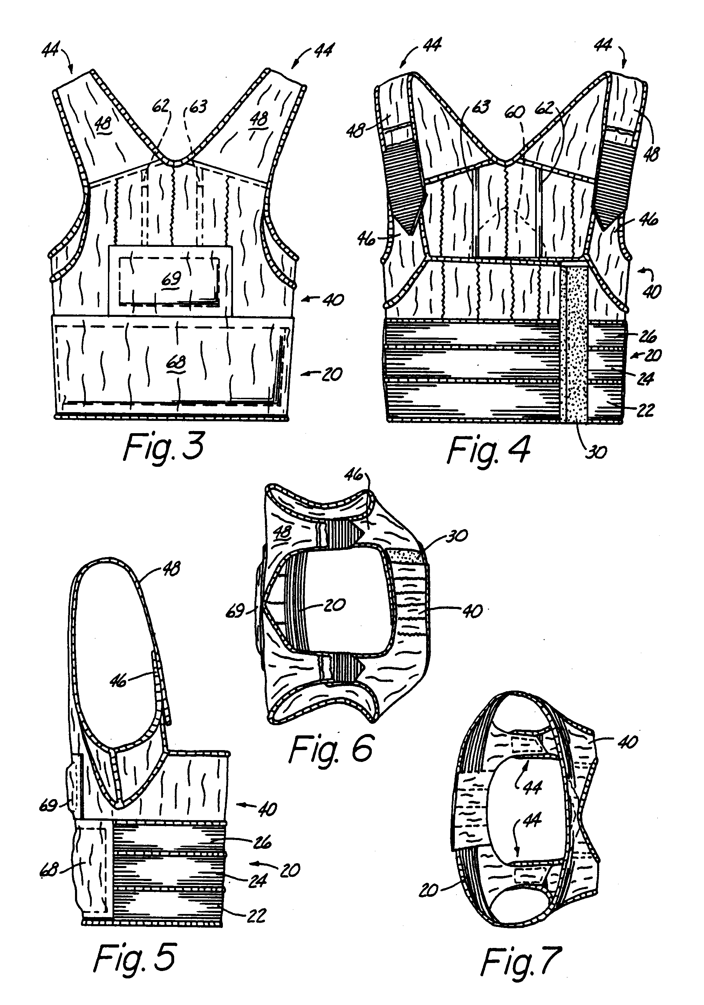

Referring now to the drawings, wherein like reference numerals designate identical or corresponding parts throughout the several views, Figure 1 shows the back support vest 10 of the present invention. The vest 10 includes a lower back supporting section 20 made of parallel panels 22, 24, 26 of elastic material joined by stitching 21. The lower panel 20 encircles the lumbar and lumbosacral region of the human body. An upper back supporting section 40 is formed of non-elastic material attached to the lower section 30. The upper section 40 encircles the body in the thoracolumbar and thoracic region. The rear of the upper section 40 extends up to the neck area while the front extends only over the abdomen up to the chest area. The front of the lower section 20 and the upper section 40 have overlapping ends that carry Velcro-type fasteners 30, 32 secured to the entire opposing surfaces of the overlapping ends to provide a large area of contact which results in a secure attachment and reliable support. A pair of selectively, adjustable shoulder straps 44 are formed by joining extensions 46, 48 by Velcro-type fasteners 50, 52.

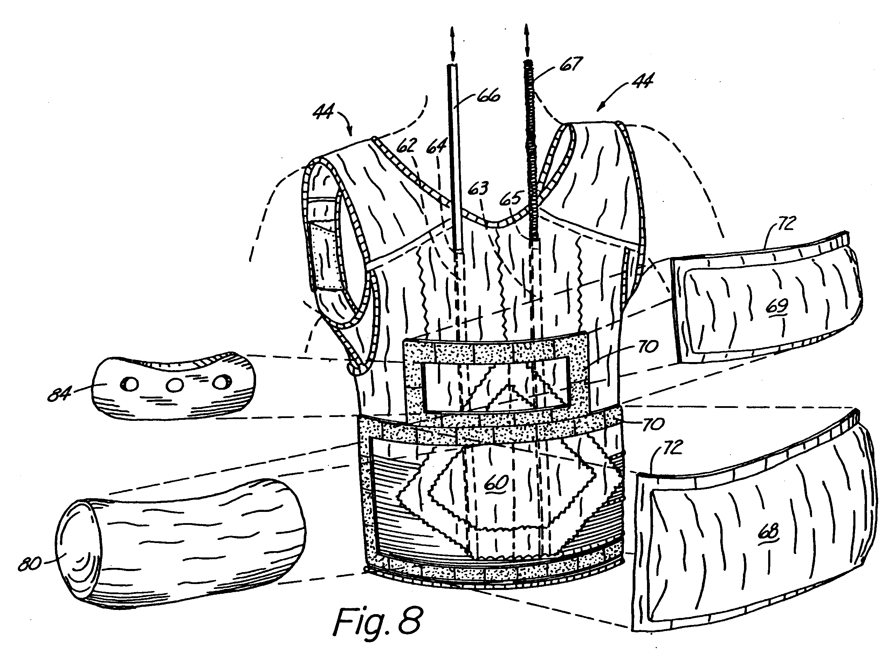

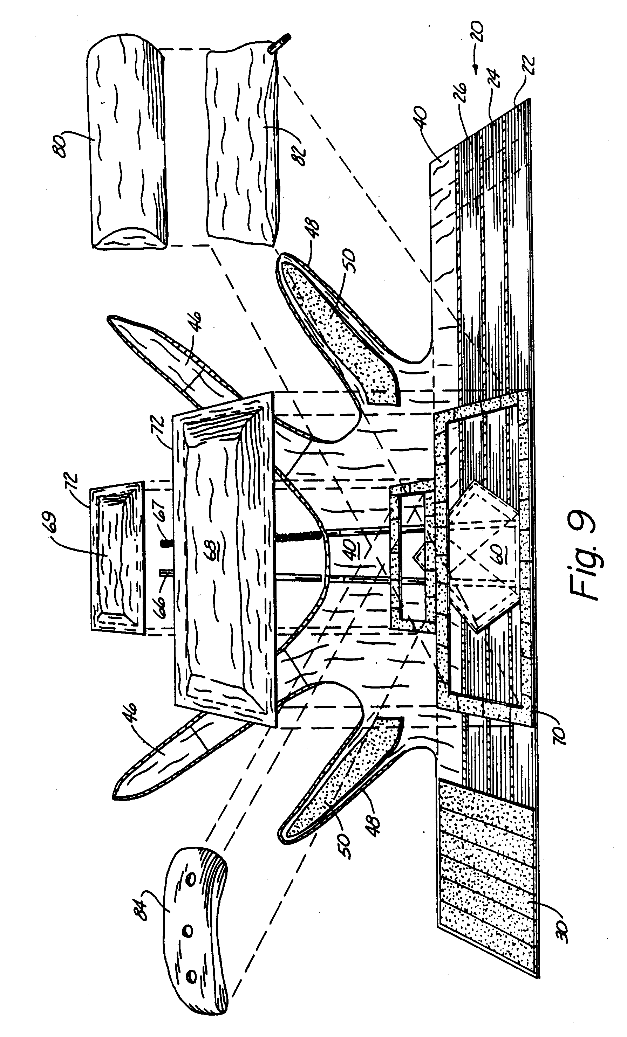

As best shown in Figures 8 and 9, a reinforced section 60 is attached by stitching to the rear of the vest 10. The reinforced section 60 is formed of a triple layer of non-elastic material that spans the lower section 20 and the upper section 40, and is centered on and extends across the dorsolumbar region. The elastic material of the lower section 20 keeps the reinforced section 60 in tension to provide effective support to the dorsolumbar region when the vest 10 is worn. A pair of sleeves 62, 63, including slit openings 64, 65 are formed in the rear of the vest 10 to receive vertical supports 66, 67 formed of bone, wire mesh, or other suitable material.

The rear of the vest 10 carries a detachable pocket 68 and an auxiliary pocket 69 attached by Velcro strips 70, 72 as illustrated in Figures 8 and 9. The pockets 68, 69 are adapted to selectively receive a number of supporting devices such as pillows 80, inflatables 82, and moulded spinal orthoses 84.

The vest 10 may be made in a number of standard sizes, e.g., small, medium, large, extra large. Also, the overlapping ends and the shoulder straps can be selectively positioned to provide a proper fit for each individual user.

When in use, the vest 10 simultaneously supports the entire back, lifts the chest, and maintains the natural curvature of the spine while the user engages in various physical activities.

Thus, it can be seen that at least all of the stated objectives have been achieved.

Obviously, many modifications and variations of the present invention are possible in light of the above teachings. It is therefore to be understood that, within the scope of the appended claims, the invention may be practiced otherwise than as specifically described.

## Claims

1. A back support vest (10), comprising
a back-supporting section (20,40) disposed to encircle the human body;
means (30,32) for releasably securing the back-supporting section in body-encircling form; and
a pair of shoulder straps (44) disposed to join front and rear lateral portions of the vest (10);
characterised in that:
the back-supporting section comprises:
a lower back-supporting section (20) of an elastic disposed to encircle the lumbar and lumbosacral region; and
an upper back-supporting section (40) of a non-elastic material attached to, and extending above, said lower section (20) and disposed to encircle the thoracolumbar and thoracic region;
and in that the vest further comprises:
a reinforced section (60) including multiple layers of non-elastic material attached to said vest and disposed to extend from the lower section (20) into the upper section (40), said reinforced section being centered on and extending across the dorsolumbar region and extending upwardly therefrom; and
a pocket (68) at the rear of said vest and disposed to extend across the lower section (20), said pocket (68) being adapted to selectively receive a support device (80;82;84).

2. A back support vest (10) according to claim 1 and further comprises an auxiliary pocket (69) at the rear of said vest above said pocket (68) and disposed to extend across the upper section (40), said auxiliary pocket (69) being adapted to selectively receive a support device (80;82;84).

3. A back support vest (10) according to claim 1 or 2 and further comprising vertical supports (66,67) attached to the rear of said vest and disposed to span the lower section (20) and upper section (40) in spaced parallel relationship to the spine.

4. A back support vest (10) according to claim 3, wherein each of said vertical supports (66,67) is carried in a sleeve (62,63) in the rear of said vest.

5. A back support vest according to any of the preceding claims, wherein said means for releasably securing the ends of the lower section (20) includes overlapping ends attachable together with a pressure sensitive contact fastener (30,32).

6. A back support vest according to any of the preceding claims, wherein said means for releasably securing ends of the upper section includes overlapping ends attachable together with a pressure sensitive contact fastener (30,32).

7. A back support vest according to claim 5, wherein said fastener (30,32) extends over the entire opposing surfaces of the overlapping ends.

8. A back support vest according to claim 6, wherein said fastener (30,32) extends over the entire opposing surfaces of the overlapping ends.

9. A back support vest according to any preceding claim, wherein said shoulder straps (44) are selectively adjustable.

10. A back support vest according to claim 8, wherein said sleeves (62,63) include slit openings (64,65) for selective removal and insertion of said vertical supports (66,67).

11. A back support vest according to any of the preceding claims, wherein said back support device is selected from a group consisting of pillows (80), inflatable (82) and moulded spinal orthoses (84).

12. A back support vest according to any of the preceding claims, wherein said pocket (68) is selectively detachable from the rear of said vest (10).

13. A back support vest according to claim 12, wherein said pocket (68) is attached with a pressure sensitive contact fastener (70,72).

14. A back support according to claim 2 and any of claims 3 to 13 when appended thereto, wherein said supporting device is a pillow (80), or an inflatable (82) or a moulded spinal orthoses (84).

15. A back support vest according to claim 13 or 14, wherein said auxiliary pocket (69) is selectively detachable from the rear of said vest (10).

16. A back support according to claim 2, or any of claims 3 to 15 when appended thereto, wherein said auxiliary pocket (69) is attached with a pressure sensitive contact fastener (70,72).

## Patentansprüche

1. Rückenstützkorsett (10), welches folgendes aufweist:
einen Rückenstützbereich (20, 40), der so angeordnet ist, daß er den menschlichen Körper umschließt;
eine Einrichtung (30, 32) zur lösbaren Befestigung des Rückenstützbereichs in einer den Körper umschließenden Form;
und ein Paar Schulterträger (44), die so angeordnet sind, daß sie vordere und hintere Seitenteile des Korsetts (10) miteinander verbinden,
dadurch gekennzeichnet,
daß der Rückenstützbereich folgendes umfaßt:
- einen unteren Rückenstützabschnitt (20) aus elastischem Material, der so angeordnet ist, daß er den Lenden- und Kreuzbeinbereich umschließt; und
- einen oberen Rückenstützabschnitt (40) aus unelastischem Material, der am unteren Stützabschnitt (20) befestigt ist und sich über diesem erstreckt, und der so angeordnet ist, daß er den Brust-/Lendenbereich und den Brustbereich umschließt;
und daß das Korsett außerdem
- einen verstärkten Bereich (60) mit mehreren Schichten aus unelastischem Material aufweist, der an dem Korsett befestigt und so angeordnet ist, daß er vom unteren Abschnitt (20) in den oberen Abschnitt (40) verläuft, wobei der verstärkte Bereich auf dem Rücken-/Lendenbereich zentriert ist und sich quer zu diesem erstreckt und von diesem aus nach oben führt,
- sowie eine Tasche (68) auf der Rückseite des Korsetts, die so angeordnet ist, daß sie quer zum unteren Abschnitt (20) verläuft, wobei sie zur bedarfsweisen Aufnahme einer Stützvorrichtung (80; 82; 84) ausgebildet ist.

2. Rückenstützkorsett (10) nach Anspruch 1,
dadurch gekennzeichnet,
daß es des weiteren eine Zusatztasche (69) auf der Rückseite des Korsetts oberhalb der Tasche (68) aufweist, die so angeordnet ist, daß sie sich quer über den oberen Abschnitt (40) erstreckt, wobei die Zusatztasche (69) zur bedarfsweisen Aufnahme einer Stützvorrichtung (80; 82; 84) ausgebildet ist.

3. Rückenstützkorsett (10) nach Anspruch 1 oder 2,
dadurch gekennzeichnet,
daß es außerdem am Rückenteil des Korsetts befestigte vertikale Stützen (66, 67) aufweist, die so angeordnet sind, daß sie den unteren Abschnitt (20) und den oberen Abschnitt (40) parallel zur Wirbelsäule im Abstand von dieser überspannen.

4. Rückenstützkorsett (10) nach Anspruch 3,
dadurch gekennzeichnet,
daß die vertikalen Stützen (66, 67) jeweils in eine Hülle bzw. einem Durchzug (62, 63) auf der Rückseite des Korsetts eingesetzt sind.

5. Rückenstützkorsett nach einem der vorhergehenden Ansprüche,
dadurch gekennzeichnet,
daß die Einrichtung zum lösbaren Befestigen der Enden des unteren Abschnitts (20) teilweise übereinandergreifende Enden aufweist, die mit einem Druckkontakt-Verschlußteil (30, 32) aneinander befestigbar sind.

6. Rückenstützkorsett nach einem der vorhergehenden Ansprüche,
dadurch gekennzeichnet,
daß die Einrichtung zum lösbaren Befestigen der Enden des oberen Abschnitts teilweise übereinandergreifende Enden aufweist, die mit einem Druckkontakt-Verschlußteil (30, 32) aneinander befestigbar sind.

7. Rückenstützkorsett nach Anspruch 5,
dadurch gekennzeichnet,
daß sich die Befestigungsmittel (30, 32) über die gesamten sich gegenüberliegenden Flächen der teilweise übereinandergreifende Enden erstrecken.

8. Rückenstützkorsett nach Anspruch 6,
dadurch gekennzeichnet,
daß sich die Befestigungsmittel (30, 32) über die gesamten sich gegenüberliegenden Flächen der teilweise übereinandergreifende Enden erstrecken.

9. Rückenstützkorsett nach einem der vorhergehenden Ansprüche,
dadurch gekennzeichnet,
daß die Schulterträger (44) nach Bedarf verstellbar sind.

10. Rückenstützkorsett nach Anspruch 8,
dadurch gekennzeichnet,
daß die Hüllen bzw. Durchzüge (62, 63) Schlitzöffnungen (64, 65) zum bedarfsweisen Entfernen und Einsetzen der vertikalen Stützen (66, 67) aufweisen.

11. Rückenstützkorsett nach einem der vorhergehenden Ansprüche,
dadurch gekennzeichnet,
daß die Rückenstützvorrichtung aus der Gruppe gewählt ist, die Polster (80), aufblasbare (82) und geformte (84) orthopädische Wirbelsäulenstützen umfaßt.

12. Rückenstützkorsett nach einem der vorhergehenden Ansprüche,
dadurch gekennzeichnet,
daß die Tasche (68) nach Bedarf vom Rückenteil des Korsetts (10) abnehmbar ist.

13. Rückenstützkorsett nach Anspruch 12,
dadurch gekennzeichnet,
daß die Tasche (68) mit einem Druckkontakt-Verschlußteil (70, 72) angebracht ist.

14. Rückenstützkorsett nach Anspruch 2 und einem der Ansprüche 3 bis 13 unter Rückbeziehung auf Anspruch 2,
dadurch gekennzeichnet,
daß die Stützvorrichtung ein Polster (80) oder eine aufblasbare (82) oder geformte (84) orthopädische Wirbelsäulenstütze ist.

15. Rückenstützkorsett nach Anspruch 13 oder 14,
dadurch gekennzeichnet,
daß die Zusatztasche (69) nach Bedarf vom Rückenteil des Korsetts (10) abnehmbar ist.

16. Rückenstützkorsett nach Anspruch 2 bzw. einem der Ansprüche 3 bis 15 unter Rückbeziehung auf Anspruch 2,
dadurch gekennzeichnet,
daß die Zusatztasche (69) mit einem Druckkontakt-Verschlußteil (70, 72) angebracht ist.

## Revendications

1. Veste de support du dos (10), comprenant:
- une section de soutien du dos (20,40) disposée de façon à entourer le corps humain;
- des moyens (30,32) pour fixer de façon amovible la section supportant le dos dans sa forme entourant le corps; et
- une paire de bretelles (44) disposées de façon à relier les parties latérales avant et arrière de la veste (10);
caractérisée en ce que la section soutenant le dos comprend:
- une section supportant le dos inférieur (20) réalisée dans un matériau élastique et disposée de façon à entourer la région lombaire et lombosacrale; et
- une section supportant le dos supérieur (40) qui y est fixée, réalisée dans un matériau non élastique, s'étendant par-dessus la section inférieure (20) et disposée de façon à entourer la région thoracolombaire et thoracique ;
et en ce que la veste comprend en outre:
- une section renforcée (60) comprenant de multiples couches de matériaux non élastiques fixées à la veste et disposée de façon à pouvoir s'étendre depuis la section inférieure (20) vers la section supérieure (40), la section renforcée étant centrée sur et s'étendant en travers de la section dorsolombaire et s'étendant vers le haut depuis celle-ci; et
- une poche (68) placée à l'arrière de la veste et disposée de façon à pouvoir s'étendre à travers la section inférieure (20), la poche (68) étant destinée à recevoir sélectivement le dispositif de soutien (80;82;84).

2. Veste de support du dos (10) selon la revendication 1, comprenant en plus une poche auxiliaire (69) placée à l'arrière de la veste au dessus de la poche (68) et conçue pour s'étendre à travers la section supérieure (40), la poche auxiliaire (69) étant destinée à recevoir sélectivement un dispositif de soutien (80;82;84).

3. Veste de support du dos (10) selon la revendication 1 ou 2, comprenant en plus des soutiens verticaux (66,67) fixés à l'arrière de la veste et destinés à atteler la section inférieure (20) et la section supérieure (40) en relation parallèle espacée par rapport à la colonne vertébrale.

4. Veste de support du dos (10) selon la revendication 3, dans laquelle chacun des soutiens verticaux (66,67) sont portés dans une nappe (62,63) à l'arrière de la veste.

5. Veste de support du dos (10) selon l'une des revendications précédentes, dans laquelle les moyens pour fixer de façon amovible les extrémités de la section inférieure (20) comprennent des extrémités se chevauchant qui peuvent être fixées ensemble avec des fermetures par contact fonctionnant à pression (30,32).

6. Veste de support du dos (10) selon l'une des revendications précédentes, dans laquelle les moyens pour fixer de façon amovible les extrémités de la partie supérieure comprennent des extrémités se chevauchant qui peuvent être fixées ensemble avec une fermeture à contact (30,32) fonctionnant à pression.

7. Veste de support du dos (10) selon la revendication 5, dans laquelle la fermeture (30,32) s'étend sur la totalité des surfaces opposées des extrémités se chevauchant

8. Veste de support du dos (10) selon la revendication 6, dans laquelle la fermeture (30,32) s'étend sur la totalité des surfaces opposées des extrémités se chevauchant.

9. Veste de support du dos (10) selon l'une des revendications précédentes, dans laquelle les bretelles (44) sont sélectivement ajustables.

10. Veste de support du dos (10) selon la revendication 8, dans laquelle les nappes (62,63) comprennent des ouvertures à encoches (64,65) pour enlever et insérer sélectivement les soutiens verticaux (66,67).

11. Veste de support du dos (10) selon l'une des revendications précédentes, dans laquelle le dispositif de soutien du dos est choisi dans le groupe comprenant les coussins (80), matelas gonflables (82) et orthoses de colonne moulées (84).

12. Veste de support du dos (10) selon l'une des revendications précédentes, dans laquelle la poche (68) est sélectivement détachable de l'arrière de la veste (10).

13. Veste de support du dos (10) selon la revendication 12, dans laquelle la poche (68) est fixée par une fermeture à contact (70,72) fonctionnant à pression.

14. Veste de support du dos (10) selon la revendication 2 et l'une des revendications 3 à 13 prise en dépendance, dans laquelle le dispositif de support est un coussin (80) ou un matelas gonflable (82) ou une orthose de colonne moulée (84).

15. Veste de support du dos (10) selon la revendication 13 ou 14, dans laquelle la poche auxiliaire (69) est sélectivement détachable de l'arrière de la veste (10).

16. Veste de support du dos (10) selon la revendication 2 ou une des revendications 3 à 15 prise en dépendance, dans laquelle la poche auxiliaire (69) est fixée par une fermeture à contact (70,72) fonctionnant à pression.
